Europäisches Patentamt

European Patent Office

Office européen des brevets

(19)

(11) Veröffentlichungsnummer : **0 490 920 B1**

(12)
# EUROPÄISCHE PATENTSCHRIFT

(45) Veröffentlichungstag der Patentschrift :
**08.12.93 Patentblatt 93/49**

(21) Anmeldenummer : **90912814.2**

(22) Anmeldetag : **06.09.90**

(86) Internationale Anmeldenummer :
**PCT/EP90/01503**

(87) Internationale Veröffentlichungsnummer :
**WO 91/03241 21.03.91 Gazette 91/07**

(51) Int. Cl.⁵ : **A61K 31/29,** A61K 33/24,
A61K 9/16, A61K 9/46,
A61K 33/08, A61K 33/12,
A23L 2/40

(54) **MAGENSÄURE BINDENDE, PHARMAZEUTISCHE ZUBEREITUNG.**

(30) Priorität : **07.09.89 CH 3260/89**
**15.09.89 CH 3366/89**
**20.02.90 CH 532/90**

(43) Veröffentlichungstag der Anmeldung :
**24.06.92 Patentblatt 92/26**

(45) Bekanntmachung des Hinweises auf die
Patenterteilung :
**08.12.93 Patentblatt 93/49**

(84) Benannte Vertragsstaaten :
**AT BE CH DE DK ES FR GB IT LI LU NL SE**

(56) Entgegenhaltungen :
**EP-A- 0 085 376**
**EP-A- 0 181 564**
**EP-A- 0 286 085**
**WO-A-88/00009**
**GB-A- 0 514 888**
**GB-A- 0 524 756**
**US-A- 4 678 661**
**US-A- 4 801 454**

(73) Patentinhaber : **Gergely, Gerhard, Dr.**
**Gartengasse 8**
**A-1050 Wien (AT)**

(72) Erfinder : **GERGELY, Gerhard**
**Gartengasse 8**
**A-1050 Wien (AT)**
Erfinder : **GERGELY, Thomas**
**Gartengasse 8**
**A-1050 Wien (AT)**
Erfinder : **GERGELY, Irmgard**
**Gartengasse 8**
**A-1050 Wien (AT)**

(74) Vertreter : **Büchel, Kurt F., Dr. et al**
**Patentbüro Dr. Büchel Letzanaweg 25**
**FL-9495 Triesen (LI)**

## Beschreibung

Die Erfindung betrifft eine pharmazeutische Zubereitung nach dem Oberbegriff des Anspruches 1.

Es sind vielerlei Systeme bekannt, die Magensäure speziell bei Hyperacidität oder bei Ulcus abpuffern, um die Angriffe der Salzsäure auf die Magenwand abzuschwächen. Die meisten Systeme leiden allerdings unter dem sogenannten "Rebound-Effekt": wenn die Magensäure abgepuffert wird, produziert der Magen zusätzliche Säuremengen und der Effekt verkehrt sich ins Gegenteil. Vor allem trifft das für Zubereitungen zu, die in Tablettenform eingenommen werden, weil hier der Einfluss lokaler Konzentrationen beim Auflösen der Tablette im Magen hinzukommt.

Hier bietet sich die Brausetablette als besondere Form eines Magenpuffers an. Das Puffersystem vieler Brausetabletten dient dazu, die Salzsäure des Magens, speziell bei Hyperacidität, zu neutralisieren. Diese Pufferkapazität wird als Säurebindungsvermögen gemessen, in mEq HCl angegeben und ist in den meisten Ländern normiert. Beispielsweise wirkt in einer herkömmlichen Puffer-Brausetablette gegen Kater nicht so sehr die ebenfalls enthaltene Acetylsalicylsäure, sondern das Puffersystem.

Nun gibt es eine Reihe von Wirksubstanzen, die zusätzlich zur Abpufferung der Magensäure einen Verlängerungseffekt, bzw. einen Schutz der Magenwand bewirken, und die in Form von Lutschtabletten, Kautabletten, aber auch als wässrige Suspension verabreicht werden.

Magenpuffersysteme waren bisher zumeist Aluminiumhydroxyd und/oder Magnesiumsalze enthaltende Kombinationen u.a.m., die als Kautabletten oder als Emulsion oder Suspension verabreicht wurden und in allen Ländern eine weite Verbreitung gefunden haben, weil die Hyperacidität eine Zivilisationskrankheit ist.

Auch hat man Magen- und Darmerkrankungen, wie z.B. Gastritis, Enteritis etc. häufig mit bismuthältigen Medikamenten behandelt. Die Bismut-Therapie hat jedoch verschiedene Nachteile:

Lösliche, auch nur kolloidal gelöste Bismutverbindungen, wie sie beispielsweise in Form von Sirup verabreicht werden, führen häufig zu unangenehmen Färbungen an den Zähnen. Das letztere gilt natürlich auch für Kau- oder Lutschtabletten, die lange Zeit im Mund- und Rachenraum verweilen.

Bismuthaltige Tabletten, insbesondere wenn sie dragiert sind, oder Kapseln, weisen zwar diesen Nachteil nicht auf, können aber zu einer lokalen Überkonzentration beim Auftreffen und Reagieren mit der Schleimhaut des Gastrointestinaltraktes führen und sind ab gewissen Mengen toxisch, insbesondere wenn sie gleich in hoher Konzentration auf bestimmte Stellen der Magen- und/oder Darmschleimhaut treffen.

Am wirksamsten scheinen Suspensionen zu sein, die auf der Basis von Magnesiumtrisilikat (insbesondere bei Gastritiden verabreicht), Sucralfat, oder auch unlöslichen Bismutsalzen aufgebaut sind. Solche Brausetabletten, die schon sehr hohe Pufferkapazität haben, haben allerdings das Problem, dass man die Wirkstoffe, welche wasserunlösliche Pulver sind, schwer in der Lösung suspendieren kann. Beim Einbringen der Tablette ins Wasser braust sie zwar; nach vollständiger Auflösung liegt aber am Boden des Glases eine dicke Schicht des Wirkstoffs, die man aufrühren müsste.

Die Erfindung hat sich nun die Aufgabe gestellt, eine pharmazeutische Zubereitung in Form eines Brausegranulates oder einer Brausetablette zu schaffen, die die genannten Nachteile vermeidet und beim Auflösen in Wasser eine wenigstens einige Minuten lang stabile Suspension der unlöslichen oder sehr wenig löslichen Wirksubstanzen oder antaciden Substanzen in Wasser erzielt. Damit sind weitere Vorteile der verbesserten Einnahme in Form einer angenehm trinkbaren Lösung wie auch einer guten Geschmacksmarkierung des Wirkstoffes und Akzeptanz verbunden.

Die Erfindung hat sich des weiteren die Aufgabe gestellt, eine Form der Darreichung von Bismutverbindungen zu schaffen, die in möglichst feinverteilter Form ohne die Bildung lokaler Überkonzentrationen an die verschiedenen Stellen der Magen- oder Darmschleimhaut gelangt. Diese Aufgaben gelingen überraschend durch die im Kennzeichen des Anspruches 1 beschriebenen Massnahmen. Weitere Ausgestaltungen der Erfindung sind in den Kennzeichen der Unteransprüche beschrieben.

Das Säurebindungsvermögen wird nach ASTM so gemessen, dass 1 g pulverisierten Wirkstoffs in 30 ml 0,1 n HCl bei Raumtemperatur 15 min gerührt wird, worauf mit 0,1 n NaOH bis auf einen pH-Wert von 3,5 zurücktitriert wird, der während 10 sec stabil bleiben muss.

Nun beschreibt zwar auch die EP-A1-286085 bereits eine magensäurebindende Zusammensetzung, bestehend aus einer in Wasser löslichen Substanz, wie Pektin- oder Zellulosederivate, die in einem sauren pH gelbindend sind, in Kombination mit einer Puffersubstanz, die im Gel dispergiert ist, wie z.B. Kasein, sowie eine säureneutralisierende Substanz, die bei Kontakt mit der Magensäure eine schaumformende oder stabilisierende Wirkung haben (Magnesiumcarbonat, Kaliumbicarbonat), wobei jedoch das Wirkprinzip ein anderes als bei der vorliegenden Erfindung ist.

Die US-A-4,801,454 beschreibt eine als fertige Suspension in den Handel gelangende, nicht brausende Zubereitung, die durch die Behandlung einer - gegebenenfalls unlöslichen - Bismutverbindung mit einem Farbstoff erzielt wird. Eine solche Bismutverbindung ohne ein Puffersystem und mit verhältnismässig hoher Konzentration weist aber die eingangs beschriebenen Nachteile auf.

In der GB-A-514,888 wird ein Verfahren beschrieben, bei dem Magnesiumhydroxid mit Natriumbicarbonat, Zitronensäure und Weinsäure gemischt werden und anschliessend zur Abspaltung des Kristallwassers der Zitronensäure auf 95-105°C erhitzt wird, wobei sich ein Teig ergibt, der anschliessend gesiebt und gegebenenfalls getrocknet wird und nach weiterem Zerkleinern zu einem Granulat, bzw. zu Tabletten verpresst wird. Wird ein solches Brausegranulat bzw. eine solche-tablette in Wasser gebracht, dann tritt ein nur langsames Brausen ein, weil das Magnesiumhydroxid aufgrund des höheren pH-Wertes die Reaktion der Brausekomponenten teilweise stört, bzw. teilweise selbst mit den sauren Bestandteilen zu löslichen Magnesiumsalzen reagiert und durch die langsame Reaktion das Magnesiumhydroxid zu Boden sinkt. Darüber hinaus bewirkt das Magnesiumhydroxid an der Magen- wand eine ebenso sofortige Neutralisation der Magensäure wie andere alkalische Substanzen und birgt damit auch wiederum die Gefahr des erwähnten Rebound-Effektes.

Die EP-A2-85376 spricht zwar von Brausesystemen mit beliebigen Wirkstoffen, nennt aber ausschliesslich agrochemische und veterinär-pharmazeutische; nichts in der Offenbarung weist den Fachmann auf die erfindungsgemässe Kombination hin, insbesondere nicht auf diejenige nach dem Kennzeichen des Anspruches 1.

Wichtig ist erfindungsgemäss, dass ein schwerlösliches, komplex gebundenes oder unlösliches und mit keinem der Brausebestandteile reagierendes Antazid bzw. ein solcher Wirkstoff, wie z.B. Magnesiumtrisilikat, Sucralfat oder ein Bismutsalz in feinstpulverisierter Form so eingesetzt werden, dass es in Mischung mit dem ebenfalls feinstpulverisierten Carbonat auf der Oberfläche einzelner Kristalle einer essbaren, organischen Säure verankert ist. Dies bringt gegenüber dem Stand der Technik gleich mehrere Vorteile auf einmal mit sich:

1. Die Brausereaktion wird nicht verzögert; die Brausetablette oder das Granulat lösen sich schnell auf und die Suspension kann getrunken werden, bevor sich die Wirkstoffteilchen abgesetzt haben.

2. Der Wirkstoff wird beim Auflösen der Tablette in einer aus Zitronensäure und den Alkalicarbonaten oder -bicarbonaten entstehenden Pufferlösung suspendiert.

3. Der Wirkstoff gelangt unreagiert und feinst verteilt an die Magenwand und bildet an dieser einen Film, der einerseits die Magensäure langsam und teils durch Gelbildung neutralisiert, andererseits seine spezifische Wirksamkeit entfaltet und somit den Rebound-Effekt verhindert.

Der besondere Vorteil solcher Systeme liegt nun darin, dass die Brausetablette nicht nur zur Suspendierung des Wirkstoffes beim Auflösen in Wasser dient, sondern durch die Alkalibicarbonate oder Erdalkalicarbonate zusätzlich eine rasche, wirksame und effektive Abpufferung der Magensäure erreicht und zudem die nicht reaktiven Wirksubstanzen, wie z.B. Magnesiumtrisilikat oder Sucralfat, die die von der Magenwand produzierte Säure langsam und sukzessive neutralisieren bzw. ihre spezifische Wirksamkeit, wie z.B. Gelbildung oder bakterizide Wirkung entfalten.

In Frage kommen dabei des weiteren so gut wie alle un- oder schwerlöslichen Bismutsalze mit pharmazeutisch zulässigen Anionen, wie z.B. Bismuthydroxid, Bismutsubnitrat, Bismutoxid, Bismutsubcarbonat, Bismutsubgallat und Bismutsubsalicylat, wie auch Mischungen dieser oder ähnlicher Bismutsalze.

Vor allem sind dadurch auch lokale Konzentrationen vermieden, wie sie sich beim Einnehmen von Tabletten oder Kautabletten ergeben, die dann auf speziellen Punkten, wo sie an der Magenwand wirken, einen besonders starken Reizeffekt entwickeln können. Dieser Reizeffekt fehlt bei der erfindungsgemässen Zubereitung völlig.

Verbessert wird das Suspensionsverhalten, wenn man zu diesen Systemen Hydrokolloide, wie beispielsweise Tragant, Guargum oder Dextrine wie Maltodextrin etc., oder ein Galactomanan hinzufügt. Sie haben die Funktion, das Wasser etwas zu beschweren, sich um die Teilchen herumzulagern und diese am Absinken oder am Koagulieren zu hindern. Baut man solche Hydrokolloide in geeigneter Weise in das System ein, dann erfolgt während des Auflösens der Brausetablette eine perfekte Suspension des wasserunlöslichen Wirkstoffes, die auch über längere Minuten hinweg stabil bleibt und bequem eingenommen werden kann.

Die in eine Mischung eingebauten Hydrokolloide verlängern im allgemeinen die Auflösezeit eines Brausesystems dadurch, indem sie die Tablette bei Zutritt von Feuchtigkeit verkleben. Andererseits können geringere Mengen als Dochtwirkung dienen, indem sie Feuchtigkeit in das Innere der Tablette einleiten. Dies sind Maltodextrine in der Grössenordnung von 1 - 5%, Gelatine oder Gummiarabicum, die zwar weniger brauchbar, aber auch in der Grössenordnung von 2-3% verwendbar sind.

Zunächst besteht die Möglichkeit, die Wirksubstanzen vermittels eines Granulats, das sowohl im normalen Verfahren, aber vorteilhafter im Vakuumverfahren aufgebaut werden kann, in das Brausesystem einzubringen, so dass während des Aufbrausens der Tablette die Substanzen automatisch suspendiert werden.

Erfindungsgemäss lassen sich solche unlösliche oder schwer lösliche Salze durch den Brauseeffekt von 2 bis 4 g schweren Brausetabletten innerhalb der Auflösungszeit gleichmässig in etwa 100 bis 150 ml Wasser suspendieren und können als wohlschmeckende Suspension eingenommen werden. Je feiner diese Teilchen sind, desto grossflächiger und vollständiger wird die Magenwand mit relativ geringen Dosen abgedeckt.

Gleichzeitig kann man mit einer entsprechenden Formulierung der Brausetablette ein Puffersystem erzeugen, das die freie Magensäure anfangs abpuffert, wodurch eine schnelle und effektive Wirkung erzielt werden kann. Bei den Bismutsalzen kommt es sehr wahrscheinlich zu einem oligodynamischen Effekt, wie er auch von Silber bekannt ist, wobei die Oligodynamik durch Spuren von Metallen oder Metallsalzen im Wasser erreicht wird. Die Metallwirksamkeit könnte z.B. für die Einwirkung des Bismut auf Campylobacter pylori verantwortlich sein. Die feinst verteilten, insbesondere unlöslichen Teilchen oder gegebenenfalls komplex gebundene Moleküle des Bismutsalzes dienen sodann zur Abdeckung der Magenwand.

Es ist nämlich von besonderem Vorteil, wenn Bismut in komplex gebundener Form vorliegt, wie z.B. in Kalium-Bismut-citrat. Komplexe, die erst bei sehr tiefem pH-Wert zerlegt werden, begünstigen die gleichmässige Verteilung im Magen-Darmtrakt und erlauben kleinste Dosierungen. Die erwähnte Kombination mit einem Puffersystem wirkt der unerwünscht raschen Zerstörung des Komplexes entgegen.

Die Erfindung lässt sich auch und gerade bei hyperaciden Personen anwenden, die relativ viel Magensäure produzieren. Möglicherweise sind die bisher beobachteten, toxischen Erscheinungen bei Bismutsalzen auf den Umstand grosser Mengen gelöster Bismutsalze, eventuell auch von gebildetem Bismutchlorid, zurückzuführen. Auch relativ grosse Mengen von Magensäure, wie sie bei Gastritiden entstehen, können durch die begleitende Pufferlösung der Brausetablette abgepuffert werden.

Weiters kann erfindungsgemäss die unerwünschte Zuführung von Natriumionen dadurch behoben werden, dass man kalium- und calciumhaltige Brausemischungen verwendet.

Geeignete Kautelen für Brause-Lösungen und ad-hoc-Suspensionen sind ein nicht übertriebener, also langsam verlaufender Brauseeffekt, damit die Teilchen sich gut verteilen können, sowie die erwähnte Anwesenheit von Kolloiden.

Die Erfindung wird im folgenden an Hand von Beispielen näher erläutert:

Beispiel 1:

140 Teile Zitronensäure der Korngrösse 0,2-0,3 mm werden in einem Mischer, vorzugsweise bereits bei 60°C, mit 2 Teilen einer konzentrierten Lösung von Mononatriumcitrat versetzt und befeuchtet. Man bringt darauf 85 Teile feines Natriumbicarbonat und lässt die Mischung anreagieren.

Sodann bringt man 50 Teile Magnesiumtrisilikat und 5 Teile Maltodextrin hinzu und lässt sie auf dem anreagierten Gemisch unter schwingendem Mischen verteilen. Man fügt dann 50 Teile Kaliumcarbonat zu und setzt die Mischungsgeschwindigkeit herab, um die körnige Struktur der Masse aufrecht zu halten.

Sodann trocknet man scharf mit einem hohen Luftstrom im Schnelltrockner oder vorzugsweise mit Anlegen hohen Vakuums in einem Vakuummischer.

Das entstehende Produkt wird zu einer Korngrösse von 0,5-1 mm gemahlen und nach Zusatz von Süssund Aromastoffen entweder als Brausegranulat verwendet oder zu Tabletten gepresst.

Beispiel 2:

140 Teile Zitronensäure, 85 Teile Natriumcarbonat, 50 Teile Magnesiumtrisilikat und 8 Teile Galactomanan werden trocken gemischt, vorzugsweise auf 60° C erhitzt, und mit einer konzentrierten Lösung von 2 Teilen Trinatriumcitrat versetzt. Die langsam beginnende Reaktion wird bis zum Ausbilden einer scholligen Struktur gemischt und sodann unmittelbar entweder durch einen heissen Luftstrom oder vorzugsweise durch Vakuumeinsatz getrocknet. Das Endprodukt wird wie in Beispiel 1 weiterverarbeitet.

Beispiel 3:

140 Teile Zitronensäure werden mit 2 Teilen einer konzentrierten Trinatriumcitratlösung befeuchtet und bei Zimmertemperatur, oder vorzugsweise bei 60°C, mit 110 Teilen Natriumbicarbonat und 100 Teilen Sucralfat vermischt. Das Sucralfat bremst die entstehende Reaktion sehr deutlich, und es kommt zur Ausbildung eines hervorragenden Granulates.

Dieses wird wie in Beispiel 1 und 2, entweder durch einen scharfen, trockenen Luftstrom, oder vorzugsweise im Vakuum getrocknet, gegebenenfalls mit einer gewünschten Menge von Süss- und/oder Aromastoffen versetzt, zu einer Korngrösse von 0,5-1 mm gesiebt und entweder als Granulat verwendet oder zu Tabletten verpresst, die 1000 mg Sucralfat pro Tablette enthalten können.

Beispiel 4:

140 Teile Zitronensäure, 85 Teile Natriumbicarbonat, 50 Teile Magnesiumtrisilikat und 20 Teile Aluminiumhydroxyd werden vermischt und mit 2 Teilen einer Mononatriumcitratlösung zur Reaktion gebracht. Nach der Reaktion fügt man 60 Teile Kaliumbicarbonat hinzu. Nach der Granulatbildung werden 10 bis 20 Teile Maltodextrin hinzugefügt, wodurch die Reaktion so gut wie zum Erliegen kommt.

Die Weiterverarbeitung des resultierenden Granulates erfolgt wie in Beispiel 1 oder 3.

Je nach Tablettengrösse lassen sich Dosierungen von 200 bis 1000 mg Magnesiumtrisilikat pro Tablette erzielen.

Bei allen Beispielen lassen sich beim Verpressen Tablettenhärten bis zu 15 kp erzielen. Bei Tablettenhärten von 10 kp ergibt sich eine Auflösungszeit von 90 bis 120 Sekunden, wobei der unlösliche Wirkstoff ohne Rückstand 5-10 Minuten in einem Getränk von etwa 100-150 ml Wasser suspendiert bleibt.

Beispiel 5:

Eine Mischung von 850 Teilen Zitronensäure, 1550 Teilen Natriumbicarbonat und 100 Teilen Tragant wird mit Alkohol oder mit Trinatriumcitratlösung granuliert, getrocknet (vakuumgetrocknet) und nach dem Trocknen mit 300 Teilen Bismutsubcarbonat vermischt.

Die Tabletten werden auf einer Tablettenpresse zu Tabletten von 2,8 g und einer Härte von 8-10 kp gepresst; sie zeigen eine Auflösungszeit von etwa 100 Sekunden in etwa 60 - 100 ml Wasser.

Beispiel 6:

850 Teile Zitronensäure, 550 Teile Natriumcarbonat, 500 Teile Calciumcarbonat und 100 Teile Maltodextrin werden gemischt, mit 50%iger Alkohol- oder Monocalciumcitratlösung granuliert, getrocknet (vakuumgetrocknet), mit 300 Teilen Bismutcarbonat versetzt und zu Tabletten gepresst.

Beispiel 7:

850 Teile Zitronensäure, 300 Teile Natriumbicarbonat, 500 Teile Kaliumcarbonat und 400 Teile Calciumcarbonat werden gemischt, mit 50%iger Alkohol- oder Pufferlösung granuliert und vakuumgetrocknet. Man fügt 300 Teile Bismutsubcitrat (komplexes Kalium-Bismut-Citrat) hinzu und presst zu Tabletten von 2,4 g. Diese Tabletten lösen sich komplett in Wasser auf und stellen eine Alternative zu den bisher üblichen löslichen Bismutsalzen dar.

Beispiel 8:

850 Teile Zitronensäure, 300 Teile Natriumbicarbonat, 500 Teile Kaliumcarbonat und 400 Teile Calciumcarbonat werden gemischt, mit einer Pufferlösung granuliert und bei 60°C bis etwa 50 mbar vakuumgetrocknet. Sodann fügt man 300 Teile des komplex gebundenen 3-Kalium-Bismut-2-Citrats hinzu und erwärmt unter Vakuum bei 70°C. Da das Komplex-Salz bis zu 8% Wasser enthalten kann, findet bei dieser Vakuumbehandlung eine Reduzierung des Wassergehaltes statt, entsprechend dem Dampfdruck des Wassers bei 60 oder 70°C. Dadurch bleibt soviel Restfeuchtigkeit übrig, dass die Auflösung des Kalium-Bismutcitrats gewährleistet ist; andererseits wird die Feuchtigkeit aber so weit reduziert, dass die nachher gepresste Brausetablette bis 50°C auch unter tropischen Konditionen stabil bleibt und keine Kettenreaktion eintritt.

Die Tabletten werden zu 2,4 g gepresst und entsprechen 120 mg $Bi_2O_3$. Diese Tabletten lösen sich klar in Wasser auf und stellen eine weitere Alternative zu den bisher üblichen löslichen Bismutzubereitungen dar.

Beispiel 9:

Je 100 ml künstlicher Magensaft (0.1 n HCl, pH 1,12) wurden mit je 1 g der zu untersuchenden Wirksubstanz versetzt. Unter Rühren wurde im Abstand von 10 sec der pH-Wert gemessen.

| Wirksubstanz | Säurebindungs-vermögen | pH-Wert nach 20 sec | 120 sec |
|---|---|---|---|
| Magnesiumoxid | 46.8 mEq/g | 9.68 | 10.18 |
| Magnesiumtrisilikat | 7.6 mEq/g | 1.15 | 1.20 |
| Bismutsubcarbonat | 2.4 mEq/g | 1.13 | 1.17 |
| Bismutsubcitrat | 3.7 mEq/g | 1.18 | 1.22 |
| Sucralfat | 15.9 mEq/g | 1.15 | 1.20 |

Im Gegensatz zu Magnesiumoxid nehmen Magnesiumtrisilikat und Sucralfat die Säure wesentlich langsamer auf. Nach 120 sec ist die Reaktion mit Magnesiumoxid beendet, wohingegen bei Magnesiumtrisilikat und Sucralfat der pH-Wert sich erst um 0.2 Einheiten verändert hat. Eine Änderung um 0.2 pH-Einheiten ist beim Magnesiumoxid schon nach 10 sec feststellbar.

## Patentansprüche

**Patentansprüche für folgende Vertragsstaaten : AT, BE, CH, DE, DK, FR, GB, IT, LI, LU, NL, SE**

1. Zubereitung aus einem - gegebenenfalls zu Tabletten verpressten - Granulat, enthaltend wenigstens einen unlöslichen, komplex gebundenen oder schwerlöslichen, Säure bindenden Stoff in Pulverform, und ein Brausesystem, das aus wenigstens einer organischen, essbaren Säure und wenigstens einem Alkali- und/oder Erdalkalicarbonat und/oder -bicarbonat besteht, dadurch gekennzeichnet, dass sie ein pharmazeutisches, magensäurebindendes Präparat ist, bei dem der Stoff ein Wirkstoff ist und jedes Granulatkorn wenigstens einen sauren Bestandteil, wenigstens einen carbonatischen Bestandteil und wenigstens einen Wirkstoff aneinander gebunden enthält, wobei der Wirkstoff in einer Menge von 5 bis 50, vorzugsweise 8 bis 30, insbesondere 12 bis 25 Gew.% vorliegt, ein Säurebindungsvermögen von 2 bis 40, vorzugsweise von 3,5 bis 25 mEq/g aufweist, mit der Säure des Brausesystems nicht reagiert und in 0,1 n HCl während 2 min den pH-Wert um höchstens 0,5 hebt, und wobei jedes Granulatkorn einen Kern als Carrier aus Kristallen der essbaren, organischen Säure, insbesondere Zitronensaure, enthält, auf dem - gegebenenfalls mit Hilfe einer Bindemittelschicht - die Wirkstoff-Pulverteilchen, bevorzugt in Mischung mit wenigstens einem Teil der carbonatischen Bestandteile in Pulverform, verankert sind.

2. Zubereitung nach Anspruch 1, dadurch gekennzeichnet, dass als Bindung der Granulatbestandteile untereinander und/oder für die Pulvermischung auf dem Kern eine Bindemittelschicht
   - aus einem Reaktionsprodukt wenigstens eines sauren mit wenigstens einem carbonatischen Bestandteil und/oder
   - aus einem Hydrokolloid, insbesondere aus wenigstens einem der Bestandteile Xanthan, Maltodextrin, Galactomanan und Tragant,
   vorgesehen ist.

3. Zubereitung nach einem der vorhergehenden Ansprüche, dadurch gekennzeichnet, dass der Wirkstoff aus wenigstens einer der folgenden Verbindungen besteht: Magnesiumtrisilikat, Sucralfat, Bismutsalz .

4. Zubereitung nach einem der vorhergehenden Ansprüche, dadurch gekennzeichnet, dass die Brausezubereitung natriumfrei oder natriumarm ist.

**Patentansprüche für folgenden Vertragsstaat : ES**

1. Verfahren zur Herstellung einer Zubereitung aus einem - gegebenenfalls zu Tabletten verpressbaren - Brausegranulat, dadurch gekennzeichnet, dass auf einen Kern als Carrier aus Kristallen einer essbaren, organischen Säure, insbesondere Zitronensäure, gegebenenfalls mit Hilfe einer Bindemittelschicht, Pulverteilchen wenigstens eines unlöslichen, komplex gebundenen oder schwerlöslichen, Magensäure bindenden Wirkstoffes, bevorzugt in Mischung mit wenigstens einem Teil carbonatischer Bestandteile eines Brausesystems in Pulverform, derart verankert werden, dass jedes Granulatkorn wenigstens einen sauren Bestandteil, wenigstens einen carbonatischen Bestandteil und wenigstens einen Wirkstoff aneinander gebunden enthält, wobei der Wirkstoff in einer Menge von 5 bis 50, vorzugsweise 8 bis 30, insbesondere 12 bis 25 Gew.% vorliegt, ein Säurebindungsvermögen von 2 bis 40, vorzugsweise von 3,5 bis 25 mEq/g aufweist, mit der Säure des Brausesystems nicht reagiert und in 0,1 n HCl während 2 min den pH-Wert um höchstens 0,5 hebt.

2. Verfahren nach Anspruch 1, dadurch gekennzeichnet, dass als Bindung der Granulatbestandteile untereinander und/oder für die Pulvermischung auf dem Kern eine Bindemittelschicht
   - aus einem Reaktionsprodukt wenigstens eines sauren mit wenigstens einem carbonatischen Bestandteil und/oder
   - aus einem Hydrokolloid, insbesondere aus wenigstens einem der Bestandteile Xanthan, Maltodextrin, Galactomanan und Tragant,
   verwendet wird.

3. Verfahren nach einem der vorhergehenden Ansprüche, dadurch gekennzeichnet, dass der Wirkstoff aus wenigstens einer der folgenden Verbindungen besteht: Magnesiumtrisilikat, Sucralfat, Bismutsalz.

4. Verfahren nach einem der vorhergehenden Ansprüche, dadurch gekennzeichnet, dass die Brausezubereitung natriumfrei oder natriumarm ist.


## Claims

### Claims for the following Contracting States : AT, BE, CH, DE, DK, FR, GB, LI, LU, NL, SE

1. A preparation of granules, possibly pressed into the form of tablets, containing at least one insoluble, complexed or not easily soluble acid-binding substance in powder form, and an effervescent system consisting of at least one organic, edible acid and at least one alkali metal and/or alkaline earth metal carbonate and/or bicarbonate, characterized as a pharmaceutical, gastric-acid-binding preparation wherein the substance is an active substance and each granule contains at least one acidic component, at least one carbonate component and at least one active substance bonded to one another, the active substance being present in an amount of 5 to 50, preferably 8 to 30, in particular 12 to 25% by weight, having an acid-binding power of 2 to 40, preferably 3,5 to 25, meq/g, not reacting with the acid of the effervescent system and increasing the pH in 0,1 N HCl during 2 min by a maximum of 0,5 wherein each granule contains a core as a carrier comprising crytals of the edible, organic acid, in particular citric acid, in which the active substance powder particles, possibly as a mixture with at least one part of the carbonate components in powder form, are anchored, eventually with the aid of a binder layer.

2. A preparation as claimed in claim 1, wherein as binder of the granuled components for binding them to one another and/or for the powder mixture on the core is provided a binder layer
   - consisting of a reaction product of at least one acidic with at least one carbonate component and/or
   - a hydrocolloid, in particular at least one of the components xanthan, maltodextrin, galactomannan and tragacanth.

3. A preparation as claimed in any of the preceding claims, wherein the active substance consists of at least one of the compounds magnesium trisilicate, sucralfate and a bismuth salt.

4. A preparation as claimed in any of the preceding claims, wherein the effervescent preparation is sodium-free or has a low sodium content.

### Claims for the following Contracting State : ES

1. A process for the manufacture of a preparation of effervescent granules, possibly capable to be pressed into the form of tablets, characterized in that powder particles of at least one insoluble, complexed or not easily soluble, gastric acid-binding active substance, preferably as a mixture with at least one part of the carbonate components in powder form, are anchored - eventually with the aid of a binder layer - on a core as a carrier comprising crystals of an edible, organic acid, in particular citric acid, in such a way that each granule contains at least one acidic component, at least one carbonate component and at least one active substance bonded to one another, the active substance being present in an amount of 5 to 50, preferably 8 to 30, in particular 12 to 25% by weight, having an acid-binding power of 2 to 40, preferably 3,5 to 25 meq/g, not reacting with the acid of the effervescent system and increasing the pH in 0,1 N HCl during 2 min by a maximum of 0,5.

2. A process as claimed in claim 1, wherein as binder of the granuled components for binding them to one another and/or for the powder mixture on the core there is provided a binder layer
  - consisting of a reaction product of at least one acidic with at least one carbonate component and/or
  - a hydrocolloid, in particular at least one of the components xanthan, maltodextrin, galactomannan and tragacanth.

3. A process as claimed in any of the preceding claims, wherein the active substance consists of at least one of the compounds magnesium trisilicate, sucralfate and a bismuth salt.

4. A process as claimed in any of the preceding claims, wherein the effervescent preparation is sodium-free or has a low sodium content.

## Revendications

### Revendications pour les Etats contractants suivants : AT, BE, CH, DE, DK, FR, GB, LI, LU, NL, SE

1. Préparation à base d'un granulat - éventuellement comprimé en des tablettes, contenant au moins une substance insoluble, liée de manière complexe ou peu soluble, capable de fixer les acides et présente sous forme de poudre, ainsi qu'un système effervescent qui consiste en au moins un acide organique comestible et en au moins un carbonate et/ou bicarbonate alcalin et/ou alcalino-terreux, préparation caractérisée en ce qu'elle est une préparation pharmaceutique, capable à fixer les acides gastriques, et dans laquelle la substance est une substance active et que chaque grain de granulat contient au moins un constituant acide, au moins un constituant de type carbonate et au moins une substance active liés les uns aux autres, la substance active étant presente en une quantité de 5 à 50, avantageusement 8 à 30, en particulier 12 à 25% en poids ayant un pouvoir de fixation d'acide de 2 à 40, avantageusement de 3,5 à 25 mEq/g, ne réagissant pas avec l'acide du système effervescent et élevant par un maximum de 0,5 la valeur du pH en 2 minutes dans HCl 0,1 N, et caractérisée en ce que chaque grain de granulat contient un noyau comme support formé de cristaux de l'acide organique comestible, notamment l'acide citrique, sur lesqueles sont ancrés, éventuellement à l'aide d'une couche de liant, les particules de poudre de substance active, préférablement en mélange avec au moins une partie des constituants de type carbonate sous forme de poudre.

2. Préparation selon la revendication 1, caractérisée en ce qu'on prévoit comme liant des constituants du granulat les uns avec les autres et/ou pour le mélange pulvérulent sur le noyau, une couche de liant formé
  - d'un produit de réaction d'au moins un constituant acide avec au moins un constituant de type carbonate et/ou
  - d'un hydrocolloide, en particulier d'au moins l'un des constituants xanthane, malto-dextrine, galactomannane, gomme adragante.

3. Préparation selon l'une des revendications précédentes, caractérisée en ce que la substance active consiste en au moins l'un des composés suivantes: trisilicate de magnésium, sucralfate et un sel de bismuth.

4. Préparation selon l'une des revendications précédentes, caractérisée en ce que le système effervescent est dépourvu de sodium ou est pauvre en sodium.

### Revendications pour l'Etat Contractant suivant : ES

1. Procédé pour la production d'une préparation à base d'un granulat effervescent - éventuellement capable à être comprimé en des tablettes - caractérisé en ce qu'on ancre sur un noyau comme support formé de cristaux de l'acide organique comestible, notamment l'acide citrique, éventuellement à l'aide d'un couche de liant, des particules de poudre au moins d'une substance active insoluble, liée de manière complexe ou peu soluble, capable de fixer les acides gastriques, préferablement en mélange avec au moins une partie des constituants de type carbonate d'un system effervescent sous forme de poudre, ans une telle manière que chaque grain de granulat contient au moins un constituant acide, au moins un constituant de type carbonate et au moins une substance active liés les uns aux autres, la substance active étant présente en une quantité de 5 à 50, avantageusement 8 à 30, en particulier 12 à 25% en poids, ayant un pouvoir de fixation d'acide de 2 à 40, avantageusement de 3,5 à 25 mEq/g, ne réagissant pas avec l'acide du système effervescent et élevant par un maximum de 0,5 la valeur du pH en 2 minutes dans HCL 0,1 N.

2. Procédé selon la revendication 1, caractérisée en ce qu'on utilise, comme liant des constituants du granulat les uns avec les autres et/ou pour le mélange pulvérulent sur le noyau, une couche de liant formé
   - d'un produit de réaction d'au moins un constituant acide avec au moins un constituant de type carbonate et/ou
   - d'un hydrocolloide, en particulier d'au moins l'un des constituants xanthane, malto-dextrine, galacto-mannane, tragacanthe.

3. Procédé selon l'une des revendications précédentes, caractérisée en ce que la substance active consiste en au moins l'un des composés suivantes: trisilicate de magnésium, sucralfate et un sel de bismuth.

4. Procédé selon l'une des revendications précédentes, caractérisée en ce que le système effervescent est dépourvu de sodium ou est pauvre en sodium.